(19) Europäisches Patentamt
European Patent Office
Office européen des brevets

(11) **EP 2 647 959 B1**

(12) # FASCICULE DE BREVET EUROPEEN

(45) Date de publication et mention de la délivrance du brevet:
**27.05.2020 Bulletin 2020/22**

(21) Numéro de dépôt: **13001065.5**

(22) Date de dépôt: **04.03.2013**

(51) Int Cl.:
*G01C 23/00* (2006.01)    *A61B 5/18* (2006.01)
*G06F 3/01* (2006.01)    *B64D 45/00* (2006.01)

(54) **Procédé et dispositif d'adaptation de l'interface homme-machine d'un aéronef selon le niveau de l'état fonctionnel du pilote**

Verfahren und Vorrichtung zur Anpassung der Mensch-Maschine-Schnittstelle eines Luftfahrzeugs je nach dem Grad des funktionellen Zustands des Piloten

Method and device for adapting the human-machine interface of an aircraft according to the level of the functional state of the pilot

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.04.2012 FR 1201003**

(43) Date de publication de la demande:
**09.10.2013 Bulletin 2013/41**

(60) Demande divisionnaire:
**20169425.4**

(73) Titulaire: **Airbus Helicopters**
**13725 Marignane Cedex (FR)**

(72) Inventeur: **Baudry, Jean-Pierre**
**83200 Toulon (FR)**

(74) Mandataire: **GPI & Associés**
**EuroParc de Pichaury**
**Bâtiment B2 - 1er Etage**
**1330, rue Guillibert de la Lauzière**
**13856 Aix-en-Provence Cedex 3 (FR)**

(56) Documents cités:
**EP-A1- 1 975 783**

• **Sandeep Mulgund ET AL: "Situation Awareness Modeling and Pilot State Estimation for Tactical Cockpit Interfaces", HCI International Conference, San Francisco, CA, 1 août 1997 (1997-08-01), XP055015781, Extrait de l'Internet: URL:http://people.brandeis.edu/~grinkus/mu lgund_rinkus_etal_97_sdpvi.pdf [extrait le 2012-01-05]**
• **CRESS J D ET AL: "An approach to developing adaptive interface technology for advanced airborne crew stations", DAYTON SECTION SYMPOSIUM, 1997., THE 14TH ANNUAL AESS/IEEE FAIRBORN, OH, USA 9 APRIL 1997, NEW YORK, NY, USA,IEEE, US, 1 janvier 1997 (1997-01-01), pages 5-10, XP010226722, DOI: 10.1109/DAYTON.1997.595089 ISBN: 978-0-7803-3965-1**
• **DURIC Z ET AL: "Integrating perceptual and cognitive modeling for adapatative and intelligent human-computer interaction", PROCEEDINGS OF THE IEEE, IEEE. NEW YORK, US, vol. 90, no. 7, 1 July 2002 (2002-07-01), pages 1272-1289, XP011065033, ISSN: 0018-9219, DOI: 10.1109/JPROC.2002.801449**

**EP 2 647 959 B1**

**Description**

La présente invention se situe dans le domaine des interfaces homme-machine des aéronefs. Elle concerne un procédé et un dispositif d'adaptation de l'interface homme-machine d'un aéronef selon le niveau de l'état fonctionnel du pilote. Ce procédé et ce dispositif sont plus particulièrement destinés aux aéronefs à voilure tournante.

Dans un aéronef, un échange permanent et bilatéral a lieu entre le pilote et l'aéronef. En effet, le pilote communique avec l'aéronef par l'intermédiaire de diverses commandes de vol ainsi que par différents moyens de saisie de données, tels qu'un clavier numérique, un désignateur, par exemple une tablette tactile permettant de désigner certains paramètres, ou différents interrupteurs. De même, l'aéronef communique de nombreuses informations au pilote, concernant notamment les conditions de vol, les paramètres de l'aéronef et l'environnement extérieur. Ces informations peuvent par exemple être communiquées visuellement au moyen de plusieurs écrans ou directement sur la visière du casque du pilote, de façon sonore par une alarme ou un dispositif de synthèse vocale ou encore de façon tactile par des vibrations au travers d'une ou plusieurs commandes de l'aéronef. De plus, l'aéronef peut également prendre en charge le pilotage de l'aéronef pour certaines phases de vol par l'intermédiaire d'un pilote automatique.

L'ensemble de ces éléments, permettant la communication entre le pilote et l'aéronef, constitue l'interface homme-machine, généralement désigné par les initiales IHM. Cette IHM permet également une communication entre le copilote de l'aéronef, quand il y en a un, et l'aéronef. Cependant, on se limitera dans la suite de cette description à la communication entre le pilote et l'aéronef, le pilote pouvant être remplacé par le copilote à tout moment.

Cette IHM permet aujourd'hui d'informer le pilote en lui fournissant l'ensemble des informations disponibles suivant les différentes phases de vol de l'aéronef et/ou les conditions de vol. Or, le pilotage des aéronefs devenant plus complexe, il est intéressant de fournir au pilote les informations requises au moment opportun. Cependant, si les facteurs humains sont pris en compte lors de la phase de conception des IHM, ce n'est pas le cas au cours du vol. En effet, la liste de ces informations et la façon de les présenter en temps-réel ne sont pas dépendantes des facteurs humains liés au pilote, tels que le stress, les émotions et la fatigue par exemple.

Pourtant, les facteurs humains sont à l'origine de nombreux incidents ou accidents survenus à des aéronefs. En effet, il est communément admis qu'une erreur de pilotage ou qu'une mauvaise application d'une procédure soit souvent imputable, tout ou partie, à une faute du pilote. Cette faute du pilote peut être causée par un ou plusieurs paramètres humains tels que le stress du pilote, sa fatigue, ses émotions ainsi que par sa charge de travail lorsqu'elle devient trop importante.

Enfin, ces facteurs humains, lorsqu'ils s'avèrent excessifs, peuvent, combinés à la complexité de certaines phases de vol, avoir une influence néfaste sur la prise de conscience par le pilote de son environnement interne, tel que la gestion des informations et des commandes de l'aéronef, ainsi que de son environnement externe, tel que le relief dans lequel évolue l'aéronef, les obstacles qu'il rencontre ou la météorologie. Cette influence néfaste peut avoir des effets non seulement sur la pertinence de la prise de décision du pilote, mais également sur les actions qu'il est amené à réaliser, notamment la qualité des manœuvres de l'aéronef.

Or, toutes les informations nécessaires sont fournies au pilote par l'IHM, et c'est aussi par l'intermédiaire de l'IHM que le pilote peut gérer ces informations. Ainsi, lorsque les facteurs humains deviennent excessifs, le pilote se trouve dans des conditions propices à la génération d'erreurs qui peuvent aboutir à un incident, voire un accident.

En particulier, les accidents imputables aux facteurs humains du pilote représentent aujourd'hui plus des trois quarts des accidents des aéronefs à voilure tournante.

L'IHM ne prend pas en compte aujourd'hui en temps-réel les facteurs humains, et plus précisément l'état fonctionnel du pilote de l'aéronef, bien que des solutions existent pour essayer d'anticiper ou de limiter leurs conséquences.

Par exemple, l'IHM dispose d'une fonction permettant de détecter, puis de corriger des erreurs simples du pilote, telles qu'un écart de trajectoire ou une erreur de saisie d'information, afin d'éviter qu'elles n'entraînent un accident. Par contre, cette fonction ne permet pas de corriger des erreurs multiples du pilote.

La sélection des pilotes permet également, lors du passage du brevet de pilotage, de tester leurs capacités, notamment vis-à-vis de la tolérance au stress et de la charge de travail qu'ils peuvent réaliser. Cependant et malgré le stress que peut générer le passage d'un examen, on ne peut pas être sûr que le caractère ou l'émotivité des pilotes ne puissent un jour avoir un effet néfaste sur la réalisation d'une manœuvre ou d'une phase de vol particulière.

Par ailleurs, les entraînements réguliers que suivent les pilotes des aéronefs, que ce soit en vol ou sur un simulateur, ainsi que les formations complémentaires qu'ils reçoivent, permettent entre autres aux pilotes d'automatiser les procédures à appliquer dans différentes conditions de vol et de compléter leurs connaissances de différents types d'aéronef. Cependant, ces entraînements et ces formations ne peuvent pas couvrir de façon exhaustive toutes les situations. De plus, les coûts élevés de ces entraînements et de ces formations peuvent également limiter leurs utilisations. Par ailleurs, le stress et la fatigue en entraînement sont toujours inférieurs à ce que le pilote rencontrera en situation réelle.

Enfin, des simulations permettent également d'évaluer la charge de travail à laquelle peut être soumis le pilote

2

suivant différentes phases de vol au cours de différentes missions réalistes. Toutefois, la liste exhaustive des différents cas qui peuvent être rencontrés par le pilote, en fonction de son niveau de connaissance et d'expérience, ne peut-être évaluée avec certitude.

On connait également le document FR2888967 qui décrit un procédé et un système de détermination d'une IHM. Après avoir défini une IHM, on réalise l'acquisition, lors d'une simulation ou d'un vol avec cette IHM, des attitudes du pilote, c'est-à-dire, par exemple, ses gestes, le parcours de son regard et ses réactions. Ensuite, après une analyse de ces attitudes, on valide l'IHM ou on l'ajuste si besoin. Ce document décrit donc la mise au point d'une IHM, mais pas son utilisation.

Par ailleurs, le document EP0292381 décrit un procédé d'élaboration d'un modèle statistique pour déterminer la charge de travail du pilote d'un aéronef. Ce procédé est basé sur des estimations par des pilotes de leur charge de travail au cours de différents vols couvrant différentes conditions de vol. Il permet ensuite une estimation de la charge du travail à laquelle est confronté un pilote en fonction des paramètres d'un vol.

On connait également le document US2007/0063854 qui décrit également un procédé d'estimation de la charge de travail du conducteur d'un véhicule. Ce procédé analyse une évaluation subjective du conducteur de sa charge de travail ainsi que des flux de données issues de divers capteurs fournissant des informations sur l'état physiologique du conducteur, l'état du véhicule et de l'environnement. L'ensemble de ces informations peuvent ensuite être traitées selon différentes techniques de modélisation telles que les réseaux bayésiens, les réseaux de neurones par exemple.

En outre, le document « Situation Awareness Modeling and Pilot State Estimation for tactical Cockpit Interfaces », du HIC International Conférence, San Francisco, CA, de août 1997, décrit une étude qui établit la faisabilité d'une interface adaptative entre un pilote et un véhicule. Une telle interface comprend principalement trois fonctions, tout d'abord déterminer la situation tactique courante de l'aéronef, puis déterminer l'état courant du pilote et enfin adapter l'interface entre le pilote et le véhicule.

Le document « An Approch to developing adaptative interface technology for advanced airborne crew stations », du Daytona Section Symposium, New-York, US, du 9 avril 1997, décrit un programme de recherche de l'« US Airforce » sur une interface adaptative entre un pilote et un aéronef. Un des points essentiels de cette étude porte sur un modèle permettant de déterminer l'état du pilote, selon principalement trois catégories d'événements : des événements externes concernant par exemple l'environnement de l'aéronef, des événements internes concernant par exemple l'état. physiologique du pilote et des événements comportementaux du pilote.

Il est à noter que l'arrière plan technologique contient le document EP 1975783 qui décrit une méthode et un système pour adapter une interface utilisateur.

Enfin, le document "Duric Z et al: "Integrating perceptual and cognitive modeling for adapatative and intelligent human-computer interaction", PROCEEDINGS OF THE IEEE, vol. 90, no. 7, 1 juillet 2002" décrit une interface adaptative (e.g. pour un cockpit d'aéronef) basée sur un modèle cognitif de l'utilisateur, prenant en compte, entre autres, son expérience et son expertise.

On constate donc qu'il existe aujourd'hui des solutions pour estimer la charge de travail d'un pilote d'aéronef, pour former ce pilote aux différentes missions qu'il aura à réaliser, ainsi que pour corriger d'éventuelles erreurs qu'il peut faire lors du pilotage. Par contre, ces solutions ne prennent toujours pas en compte les facteurs humains en temps-réel et ne s'adaptent donc pas au niveau de l'état fonctionnel du pilote tout au long de la mission. De plus, ces solutions ne prennent pas en compte l'état fonctionnel initial du pilote avant le démarrage de la mission. On entend par niveau de l'état fonctionnel du pilote l'ensemble de ses capacités tant physiques, physiologiques que psychologiques lui permettant de réaliser efficacement ou non les tâches auxquelles il est confronté. On utilisera par la suite l'expression « niveau fonctionnel » pour désigner plus simplement le niveau de l'état fonctionnel du pilote.

Les tâches du pilote correspondent à la prise de conscience de la situation courante, aux décisions à prendre et aux actions que doit réaliser le pilote lors d'une phase de vol. Il peut s'agir de tâches plus ou moins complexes, telles que des tâches de pilotage afin d'assurer un atterrissage, ou des tâches de navigation telles que rentrer les coordonnées d'un point de passage. De plus, ces tâches peuvent être rendues plus difficiles à appréhender par le pilote notamment lors de contraintes temporelles fortes.

Par exemple, il peut s'agir de réaliser un atterrissage proche d'obstacles et par mauvaise visibilité ou bien de redéfinir complètement une route à la suite d'une fuite carburant tout en tenant compte de conditions météorologiques défavorables.

La présente invention a alors pour objet de proposer un procédé, selon la revendication 1, permettant de prendre en compte, de façon automatique et en temps-réel, ce niveau fonctionnel du pilote et d'adapter, au cours du vol, l'IHM de l'aéronef selon ce niveau fonctionnel du pilote. Ce procédé d'adaptation de l'IHM d'un aéronef vise donc non seulement à anticiper l'assistance à apporter au pilote mais également à l'optimiser.

Le procédé d'adaptation d'une IHM d'un aéronef selon le niveau fonctionnel d'un pilote comporte plusieurs étapes successives. Tout d'abord, on détermine, avant le démarrage d'une mission, les caractéristiques de cette mission ainsi que les caractéristiques et l'état physiologique initial du pilote. Ensuite, au cours de la mission, on détermine l'avancement de la mission et un état courant de l'aéronef ainsi qu'un comportement courant du pilote. Puis, on détermine un niveau fonctionnel courant du pilote. Par la suite, on détermine une valeur de comparaison en fonction de ce niveau fonctionnel courant du pilote et on la compare avec au moins un niveau fonctionnel de référence. Enfin, on adapte l'IHM de l'aéronef en fonction de cette valeur de comparaison et de chaque niveau fonctionnel de référence.

On entend par adapter l'IHM le fait, par exemple, de modifier cette IHM au niveau de l'affichage des divers informations disponibles ou bien de réaliser des actions par l'intermédiaire du pilote automatique de l'aéronef. Cette adaptation a pour but d'aider, d'assister voire de suppléer le pilote dans la situation courante.

Pour pouvoir déterminer le niveau fonctionnel courant du pilote, il faut préalablement connaître les caractéristiques de la mission à réaliser, notamment les tâches qu'elle comporte et la difficulté de ces tâches. Il faut également connaître les caractéristiques du pilote, notamment ses connaissances et ses expériences vis-à-vis de ces différentes tâches, ainsi que les réactions, liées à son caractère, qu'il peut avoir face à ces tâches.

De plus, il faut connaître le comportement courant du pilote et le changement de ce comportement au cours de la mission. Ce comportement courant du pilote est notamment caractérisé par son état physiologique courant.

L'état physiologique du pilote est obtenu par différentes mesures réalisées sur le pilote par des capteurs physiologiques. Ces mesures permettent de connaître, entre autres et à chaque instant le rythme cardiaque du pilote, l'activité de son cerveau, la cadence de sa respiration, la dilatation de ces pupilles, le clignement et le mouvement de ses yeux, son niveau de transpiration, le mouvement de sa tête, ses modifications faciales telles qu'un sourire ou une grimace, la tension de ses muscles. Les différents capteurs physiologiques nécessaires à ces mesures sont connus et classiquement utilisés dans ce but.

Par contre, l'état physiologique du pilote n'est pas constant et peut varier chaque jour, en fonction de son humeur, son état de santé ainsi que son environnement extérieur tel que la température ambiante. De plus, les mesures réalisées par des capteurs physiologiques pouvant varier dans le temps, chaque capteur physiologique utilisé doit donc être étalonné régulièrement.

Il est donc nécessaire de réaliser des mesures avant le démarrage de chaque mission, le pilote étant au repos, afin de déterminer l'état physiologique initial du pilote : il est considéré au repos lorsqu'il est assis à son poste de pilotage et ne réalise aucune tâche.

Ensuite, l'état physiologique courant du pilote au cours de la mission est déterminé par ces mêmes capteurs physiologiques. Le procédé selon l'invention peut ainsi déterminer une évolution de l'état physiologique du pilote entre son état physiologique initial et son état physiologique courant, permettant d'estimer en partie un changement de son comportement.

Par ailleurs, il faut connaître aussi l'avancement de la mission, c'est-à-dire la phase de vol courante dans laquelle se trouve l'aéronef et les tâches courantes que doit alors réaliser le pilote, ainsi que l'état courant de l'aéronef. Cet état courant de l'aéronef, qui comprend à la fois des paramètres fonctionnels de l'aéronef, au niveau des moteurs par exemple, que des paramètres de vol tels que la vitesse, l'altitude est obtenu par l'intermédiaire de différents capteurs classiquement présents dans un aéronef.

Ainsi, le principe de l'invention repose sur différentes données d'entrée, à la fois sur des caractéristiques constantes déterminées une fois pour toute, avant la mission, et sur des mesures objectives réalisées avant la mission pour l'état physiologique initial du pilote, et au cours de la mission pour son état physiologique courant, l'avancement de la mission et l'état courant de l'aéronef.

Ensuite, on détermine un niveau fonctionnel courant du pilote, au cours de la mission, en prenant en compte ces différentes données d'entrée.

Pour réaliser ce calcul, une technique issue de l'intelligence artificielle, telle que des systèmes neuronaux à apprentissage, peut être employée. Cette technique utilise par exemple des évaluations faites par des pilotes, lors de simulation ou de vols précédents, sur leurs niveaux fonctionnels courants en fonction de différentes missions et différentes conditions de vol. Un algorithme utilise alors ces évaluations, avec les données des différentes missions et conditions de vol dans lesquelles elles ont été obtenues, et les données d'entrée précédemment citées afin d'obtenir une valeur entière caractérisant le niveau fonctionnel courant du pilote.

Ce calcul du niveau fonctionnel courant du pilote correspond, de fait, aux capacités du pilote à réaliser les tâches courantes au cours de la mission en fonction de son expérience, de ces connaissances et de son caractère ainsi que du changement de son comportement. Ce calcul est pondéré en fonction de la phase de vol courante, des difficultés des tâches courantes de la mission et de l'état courant de l'aéronef.

Dans ce calcul, la fréquence courante des erreurs commises par le pilote et identifiées par l'IHM peut également être prise en compte. En effet, cette fréquence d'erreur peut, par exemple, mettre en évidence un état de stress important du pilote ou bien une charge de travail trop importante, caractérisant ainsi son comportement courant.

Une valeur de comparaison peut alors être déterminée en fonction de ce niveau fonctionnel courant du pilote et ensuite être comparée à au moins un niveau fonctionnel de référence. Un niveau fonctionnel de référence correspond à un niveau fonctionnel limite à partir duquel le pilote ne peut plus prendre pleinement conscience de la situation ou bien prendre une décision adéquate ou encore assurer efficacement la totalité des actions à réaliser. Une aide doit alors lui être apportée afin de lui permettre d'assurer la continuité de la mission avec le maximum de sécurité. Cette aide lui est alors fournie par une adaptation de l'IHM, au minimum via la présentation des informations appropriées, voire par la réalisation, de tout ou partie, de certaines tâches.

Chaque niveau fonctionnel de référence est constant quelle que soient la mission et l'aéronef. Il est indépendant du pilote.

Le procédé d'adaptation est dans ce cas amené à assister voire suppléer le pilote selon la valeur de comparaison, qui est fonction de son niveau fonctionnel courant. L'invention permet ainsi d'assister de façon automatique et optimale le pilote dans sa prise de décision lorsqu'il évolue par exemple vers un état de stress ou de charge de travail élevée, voire vers une incapacité totale de faire face à la situation.

Les caractéristiques de la mission comprennent l'ensemble des différentes phases de vol de la mission à réaliser et les tâches associées ainsi que le niveau de difficulté de chaque phase de vol et de chaque tâche. Toute mission peut en effet être fractionnée en différentes phases de vol, telles que le décollage ou le vol de croisière ou l'atterrissage, en prenant en compte son environnement. Par exemple, un atterrissage sur une piste d'aérodrome et un atterrissage sur une plateforme pétrolière en haute mer sont deux phases de vol distinctes.

De plus, chaque phase de vol comporte différentes tâches à réaliser, telles que des tâches de pilotage, de gestion de l'aéronef ou de navigation. Enfin, à chaque phase de vol et chaque tâche peut être associé un niveau de difficulté, un atterrissage sur une plateforme pétrolière en haute mer étant à priori plus complexe, donc avec un niveau de difficulté supérieur, qu'un atterrissage sur une piste d'aérodrome. L'ensemble des différentes phases de vol et des tâches associées ainsi que leurs niveaux de difficulté sont établis au préalable à la mission et une fois pour toute. Ils sont par exemple stockés dans une première base de données dédiée.

La mission à réaliser doit alors être décomposée en différentes phases de vol correspondant aux phases répertoriées dans la base de données avec les niveaux de difficulté associés.

Les caractéristiques du pilote comprennent son niveau d'expérience et ses connaissances lui permettant de réaliser les différentes tâches de la mission ainsi que son état psychologique. Cet état psychologique, directement lié à son caractère, correspond notamment aux réactions qu'il peut avoir face à ces différentes tâches et suivant son niveau de stress par exemple. Il peut être établi suite à différents questionnaires psychologiques adaptés, afin de déterminer les traits dominants de son caractère, son émotivité, son impulsivité par exemple.

Par ailleurs, le niveau d'expérience du pilote concerne aussi bien son expérience dans le pilotage de différents types d'aéronefs que dans la réalisation de diverses missions, en intégrant sa pratique des phases de vol répertoriées dans la première base de données.

Cet état psychologique du pilote ainsi que ses connaissances et son expérience étant peu susceptibles d'évoluer rapidement avec le temps, ils sont établis au préalable à la mission. Ils sont par exemple stockés dans une seconde base de données dédiée. Cependant, le retour d'expérience des missions auxquelles le pilote participe peut permettre d'alimenter cette seconde base de données en intégrant les réactions dont il a fait preuve pour compléter son état psychologique ainsi que son expérience.

Le comportement courant du pilote au cours de la mission comprend son état physiologique courant ainsi qu'une fréquence courante des erreurs du pilote. En effet, l'évolution de l'état physiologique du pilote permet d'estimer en partie le changement de son comportement. Par exemple, une augmentation de sa transpiration ou de son rythme cardiaque est un signe d'augmentation de son stress. De même, des mouvements rapides de va-et-vient des yeux peuvent également mettre en évidence une situation de stress ou d'anxiété, comme des clignements fréquents de ses yeux révèlent une fatigue du pilote. De fait, en comparant l'état physiologique courant du pilote avec son état physiologique initial, on peut déterminer un changement de son comportement.

De plus, le nombre d'erreurs commises par le pilote est également un signe que la charge de travail à laquelle il doit faire face ou que la complexité des tâches à réaliser est trop importante par rapport à ses capacités courantes, par exemple à cause d'une fatigue ou d'un stress important. L'IHM permet de détecter et éventuellement de corriger des erreurs simples, telle que des écarts intempestifs par rapport à une trajectoire à suivre ou encore une erreur de saisie de paramètres hors d'un intervalle de définition. Par suite, en combinant l'état physiologique courant du pilote et la fréquence courante de ses erreurs, on peut déterminer le changement de son comportement au cours de la mission.

Dans un exemple, la valeur de comparaison est déterminée afin qu'elle soit égale au niveau fonctionnel courant du pilote. De fait, le procédé peut comparer ce niveau fonctionnel courant du pilote avec chaque niveau fonctionne! de référence et adapter l'IHM en conséquence.

De plus, selon l'invention, on conserve un historique des niveaux fonctionnels du pilote tout au cours d'une mission afin de pouvoir extrapoler en temps réel le niveau fonctionnel du pilote. Pour cela, on enregistre le niveau fonctionnel courant du pilote dans un historique des niveaux fonctionnels constituant une troisième base de données dédiée.

Par exemple, cet historique est constitué par les niveaux fonctionnels successifs du pilote sur les cinq dernières secondes. Cette durée de l'historique peut être fonction de la phase de vol, telle qu'un vol de croisière ou un atterrissage, ou bien des conditions de vol.

Selon l'invention, à partir de cet historique des niveaux fonctionnels, on calcule une tendance de l'évolution du niveau fonctionnel du pilote et on détermine ensuite un niveau fonctionnel prédictif du pilote.

La valeur de comparaison est dans ce cas déterminée afin qu'elle soit égale au niveau fonctionnel prédictif du pilote. Ainsi, le procédé peut comparer ce niveau fonctionnel prédictif du pilote avec chaque niveau fonctionnel de référence, réagissant de façon adéquate et automatiquement afin d'assister par anticipation et au mieux le pilote.

En effet, une originalité importante de l'invention réside dans le fait, qu'à partir de quelques itérations du calcul du niveau fonctionnel courant du pilote, le procédé est capable de dégager une tendance de l'évolution de ce niveau fonctionnel du pilote. A partir de cet historique, on dispose des couples de valeurs $(NFP_i, t_i)$ représentant le niveau fonctionnel du pilote $NFP_i$ à l'instant $t_i$. Ensuite, on peut déterminer une tendance telle qu'une droite, définie par $T = a \times t + b$, soit la plus proche de l'ensemble des points $(NFP_i, t_i)$ selon un graphe sur lequel le temps $t_i$ est en abscisse et le niveau fonctionnel $NFP_i$ en ordonnée.

Cette droite se caractérise par le fait qu'elle minimise la somme des distances au carré des points $(NFP_i, t_i)$ à cette droite, c'est-à-dire que la fonction $F(a,b) = \sum_{1}^{n} (NFP_i - a \times t_i - b)^2$ est minimum, $n$ étant le nombre d'itération(s) à prendre en compte et $t_n$ le temps pour lequel a été déterminé le dernier niveau fonctionnel du pilote $NFP_n$. Ce nombre $n$ d'itération dépend à la fois de la fréquence de mesure du niveau fonctionnel courant du pilote $NFP_i$ et de la durée sur laquelle est stocké l'historique de ces niveaux fonctionnels du pilote $NFP_i$.

De plus, on peut définir un couple de valeurs moyennes $(NFP_{moyen}, t_{moyen})$ sur l'intervalle de temps de $t_i$ à $t_n$,

tel que $NFP_{moyen} = \dfrac{\sum_{i=1}^{n} NFP_i}{n}$ et $t_{moyen} = \dfrac{\sum_{i=1}^{n} t_i}{n}$.

Cette droite passe obligatoirement par ce couple $(NFP_{moyen}, t_{moyen})$. De fait, on a $NFP_{moyen} = a \times t_{moyen} + b$.

On en déduit ensuite $b = NFP_{moyen} - a \times t_{moyen}$.

Par suite, le coefficient directeur de la droite correspondant à la tendance du niveau fonctionnel du pilote est :

$$a = \frac{\sum_{i=1}^{n} \left[ (t_i - t_{moyen}) \times (NFP_i - NFP_{moyen}) \right]}{\sum_{i=1}^{n} (t_i - t_{moyen})^2}.$$

Une fois la tendance de l'évolution du niveau fonctionnel du pilote définie, on peut estimer un niveau fonctionnel prédictif du pilote $NFPP$ selon la formule $NFPP = a \times (t_n + \Delta t) + b$, avec $\Delta t$ représentant la variation de temps à partir du temps $t_n$. La valeur de $\Delta t$ peut être de l'ordre d'une seconde à quelques secondes.

L'IHM de l'aéronef comporte différents systèmes permettant au pilote de communiquer avec l'aéronef et à l'aéronef de communiquer avec la pilote.

Par exemple, pour permettre à l'aéronef de communiquer avec le pilote, l'IHM peut comporter au moins un système d'affichage d'informations, tel un écran ou la visière du casque du pilote, au moins un système d'information sonore ou au moins un système d'information tactile. De plus, l'IHM peut également comporter au moins un système de pilotage automatique de l'aéronef, capable de suppléer le pilote pour suivre une trajectoire prédéterminée, voire pour réaliser certaines manœuvres.

Selon un mode de réalisation de l'invention, le procédé d'adaptation comporte trois niveaux fonctionnels de référence et agit sur l'IHM de l'aéronef en fonction de la valeur de comparaison et de ces trois niveaux fonctionnels de référence.

Tout d'abord, si la valeur de comparaison est inférieure aux trois niveaux fonctionnels de référence, aucune adaptation de l'IHM n'est nécessaire. Par suite, aucune modification de l'IHM n'est effectuée, le pilote étant pleinement apte à réaliser les différentes tâches courantes de la phase de vol courante. Le procédé redémarre alors un cycle afin de déterminer une nouvelle valeur de comparaison.

Par contre, si la valeur de comparaison est égale ou supérieure à un premier niveau fonctionnel de référence et inférieure à un second niveau fonctionnel de référence, une adaptation de l'IHM est nécessaire afin d'améliorer la prise de conscience de la situation par le pilote. Dans ce cas, au moins une modification de la présentation des informations fournies au pilote est effectuée. Le but de cette modification est de sensibiliser le pilote à des informations particulières sur lesquelles il doit focaliser son attention. Par exemple, le nombre, la taille et/ou la forme des informations fournies au pilote, qu'elles soient visuelles, sonores ou tactiles, sont modifiés. Le procédé redémarre ensuite un cycle afin de déterminer une nouvelle valeur de comparaison.

De plus, si la valeur de comparaison est égale ou supérieure à un second niveau fonctionnel de référence et inférieure à un troisième niveau fonctionnel de référence, une adaptation de l'IHM est nécessaire afin d'assister le pilote dans sa prise de décision. Dans ce cas, une proposition de solution au problème courant à résoudre est indiquée au pilote. Le but est de fournir au pilote une solution au problème détecté, tout en lui laissant les commandes de l'aéronef afin d'appliquer cette solution ou une autre solution qu'il jugerait plus adaptée. Par exemple, un écran de l'IHM affiche une route sécurisée à suivre pour atteindre une zone d'atterrissage adéquate à la suite d'un problème important tel qu'un feu de moteur. Le procédé redémarre ensuite un cycle afin de déterminer une nouvelle valeur de comparaison.

Enfin, si la valeur de comparaison est égale ou supérieure à un troisième niveau fonctionnel de référence, une adaptation autoritaire de l'IHM est indispensable pour agir à la place du pilote. Dans ce cas, une reprise automatique d'autorité par l'aéronef au détriment du pilote est engagée. Dans ce cas, le pilote semble dépassé par la situation et incapable de trouver une solution et même d'appliquer une solution que pourrait lui proposer l'IHM. Il est donc plus sûr pour garantir la sécurité de l'aéronef et des personnes à bord de placer l'aéronef en pilotage automatique ou de corriger une manœuvre mettant en péril cette sécurité. Par exemple, le pilotage automatique de l'aéronef est activé automatiquement pour remettre la machine dans un état de sécurité, tel que rester en vol stationnaire ou suivre une trajectoire prédéterminée. Le procédé redémarre ensuite un cycle afin de déterminer une nouvelle valeur de comparaison.

La présente invention a aussi pour objet un dispositif, selon la revendication 9, d'adaptation d'une IHM d'un aéronef selon le niveau fonctionnel d'un pilote. Un tel dispositif comprend un ensemble de capteurs physiologiques, au moins un système scrutateur, au moins deux bases de données et au moins une unité de traitement.

L'ensemble de capteurs physiologiques mesure un état physiologique du pilote, le système scrutateur contrôlant les actions de ce pilote afin de détecter les éventuelles erreurs qu'il effectuerait. De plus, une première base de données contient les caractéristiques de différentes missions réalisables et une seconde base de données contient les caractéristiques du pilote. Par ailleurs, l'IHM de l'aéronef comporte au moins un système d'affichage d'informations, au moins un système d'information sonore, au moins un système d'information tactile ou au moins un système de pilotage automatique de l'aéronef.

Au cours d'une mission, l'unité de traitement permet de déterminer un niveau fonctionnel courant du pilote en interprétant les caractéristiques de la mission, les caractéristiques du pilote, l'état physiologique initial du pilote avant le démarrage de la mission, l'état physiologique courant du pilote au cours de la mission, la fréquence courante des erreurs effectuées par le pilote, l'avancement de la mission et l'état courant de l'aéronef. Cet état courant de l'aéronef est obtenu par l'intermédiaire de différents capteurs présents dans l'aéronef. L'unité de traitement détermine ensuite une valeur de comparaison en fonction de ce niveau fonctionnel courant du pilote puis la compare avec au moins un niveau fonctionnel de référence. Enfin, l'unité de traitement agit sur l'IHM de l'aéronef en fonction de la valeur de comparaison et de chaque niveau fonctionnel de référence.

Dans un exemple, la valeur de comparaison est égale au niveau fonctionnel courant du pilote.

Selon l'invention, les niveaux fonctionnels du pilote sont stockés dans une troisième base de données présente dans l'aéronef et constituant ainsi un historique des niveaux fonctionnels. L'unité de traitement est configurée pour calculer une tendance de l'évolution du niveau fonctionnel du pilote à partir de cet historique, puis déterminer un niveau fonctionnel prédictif du pilote. La valeur de comparaison est égale à ce niveau fonctionnel prédictif du pilote. Les formules précédemment évoquées permettent de déterminer ce niveau fonctionnel prédictif du pilote.

L'unité de traitement comporte trois niveaux fonctionnels de référence et agit sur l'IHM de l'aéronef de différentes manières en fonction de la valeur de comparaison et des trois niveaux fonctionnels de référence.

Si la valeur de comparaison est inférieure aux niveaux fonctionnels de référence, aucune adaptation de l'IHM n'est nécessaire. Par suite, aucune modification automatique de l'IHM n'est effectuée.

Si la valeur de comparaison est égale ou supérieure à un premier niveau fonctionnel de référence et inférieure à un second niveau fonctionnel de référence, une adaptation de l'IHM est nécessaire afin d'améliorer la prise de conscience de la situation par le pilote. Dans ce cas, au moins une modification des informations fournies au pilote est effectuée.

Si la valeur de comparaison est égale ou supérieure à un second niveau fonctionnel de référence et inférieure à un troisième niveau fonctionnel de référence, une adaptation de l'IHM est nécessaire afin d'assister le pilote dans sa prise de décision. Dans ce cas, une proposition de solution au problème courant à résoudre est indiquée au pilote.

Si la valeur de comparaison est égale ou supérieure à un troisième niveau fonctionnel de référence, une adaptation autoritaire de l'IHM est indispensable pour agir à la place du pilote. Dans ce cas, une reprise automatique d'autorité par l'aéronef au détriment du pilote est engagée.

L'invention et ses avantages apparaîtront avec plus de détails dans le cadre de la description qui suit avec des exemples de réalisation donnés à titre illustratif en référence aux figures annexées qui représentent :

- la figure 1, un schéma synoptique du procédé selon l'invention,

- la figure 2, un graphe représentant les niveaux fonctionnels du pilote de l'aéronef, et

- les figures 3 et 4, un mode de réalisation du dispositif selon l'invention.

Les éléments présents dans plusieurs figures distinctes sont affectés d'une seule et même référence.

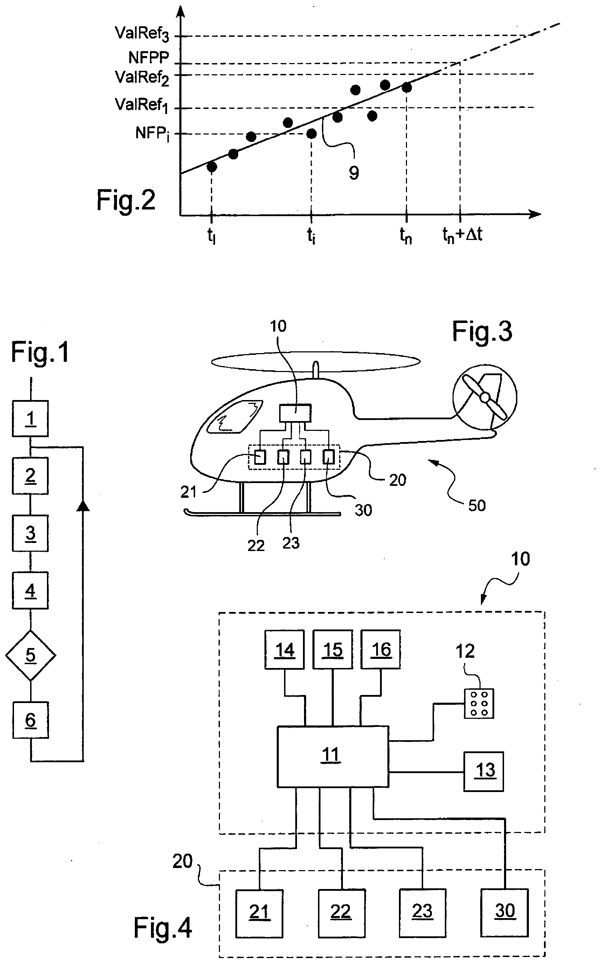

La figure 1 représente le schéma synoptique du procédé d'adaptation d'une interface homme-machine 20 d'un aéronef 50 selon le niveau fonctionnel d'un pilote comportant six étapes successives.

Au cours d'une première étape 1, on détermine, avant le démarrage d'une mission, les caractéristiques de cette mission ainsi que les caractéristiques et l'état physiologique initial du pilote. Ensuite pendant une seconde étape 2, au cours de la mission, on détermine l'avancement de la mission et un état courant de l'aéronef 50 ainsi qu'un comportement courant du pilote. Puis, au cours d'une troisième étape 3, on détermine un niveau fonctionnel courant du pilote *NFCP* et lors d'une quatrième étape 4, on détermine une valeur de comparaison *VC* en fonction du niveau fonctionnel courant du pilote *NFCP*. Au cours d'une cinquième étape 5, on compare cette valeur de comparaison *VC* avec trois niveaux fonctionnels de référence *ValRef1, ValRef2* et *ValRef3*. Enfin, au cours d'une sixième étape 6, on agit sur l'IHM 20 de l'aéronef 50 en fonction de cette valeur de comparaison *VC* et des trois niveaux fonctionnels de référence *ValRef1, ValRef2* et *ValRef3*.

Les niveaux fonctionnels de référence *ValRef1, ValRef2* et *ValRef3* sont constants quels que soient la mission et l'aéronef. Ils sont définis afin que *ValRef1* soit inférieur à *ValRef2* et que *ValRef2* soit inférieur à *ValRef3*.

Les figures 3 et 4 représentent un mode de réalisation du dispositif d'adaptation 10 d'une IHM 20 implanté dans un aéronef 50. Le dispositif 10 comprend un ensemble de capteurs physiologiques 12, un système scrutateur 13, trois bases de données 14, 15 et 16 ainsi qu'une unité de traitement 11.

L'ensemble de capteurs physiologiques 12 mesure un état physiologique du pilote, le système scrutateur 13 contrôlant les actions de ce pilote afin de détecter les éventuelles erreurs qu'il effectuerait. De plus, une première base de données 14 contient les caractéristiques de différentes missions réalisables et une seconde base de données 15 contient les caractéristiques du pilote. Par ailleurs, l'IHM 20 de l'aéronef 50 comporte un système d'affichage d'informations 21, un système d'information sonore 22, un système d'information tactile 23 et un système de pilotage automatique 30 de l'aéronef 50. La troisième base de données 16 contient l'historique des niveaux fonctionnels.

Dans ces conditions, lors de la première étape 1 du procédé selon l'invention, la mission est fractionnée en différentes phases de vol dont les caractéristiques sont connues et stockées dans la première base de données 14. On détermine ainsi les différentes tâches à réaliser dans ces phases de vol et les difficultés associées. De plus, la seconde base de données contient les caractéristiques du pilote, c'est-à-dire son expérience et ses connaissances ainsi que son état psychologique. Enfin, le procédé permet de déterminer, par l'intermédiaire de l'ensemble de capteurs physiologiques 12, l'état physiologique initial du pilote avant le démarrage de la mission, le pilote étant en repos.

Lors de la seconde étape 2, au cours de la mission, on détermine l'avancement de la mission, c'est-à-dire la phase de vol dans laquelle se trouve l'aéronef 50 et un état courant de l'aéronef 50, c'est-à-dire les paramètres fonctionnels et de vol de l'aéronef 50. L'état courant de l'aéronef 50 est obtenu par l'intermédiaire de différents capteurs présents dans l'aéronef 50. On détermine également un comportement courant du pilote qui comprend son état physiologique courant, obtenu par l'intermédiaire de l'ensemble de capteurs physiologiques 12 et la fréquence courante d'erreurs du pilote obtenue par le système scrutateur 13.

Puis, en troisième étape 3, l'unité de traitement 11 détermine un niveau fonctionnel courant du pilote en fonction des informations déterminées ou mesurées aux première et seconde étapes 1, 2. L'unité de traitement 11 utilise par exemple un modèle d'intelligence artificielle tel que des systèmes neuronaux à apprentissage permettant d'interpréter l'ensemble de ces informations. Le niveau fonctionnel courant du pilote ainsi obtenu correspond en fait aux capacités du pilote à réaliser les tâches courantes de la mission en fonction de son expérience, de ces connaissances et de son caractère ainsi que de son comportement courant, c'est-à-dire l'évolution de son état physiologique et du nombre d'erreurs qu'il réalise. Ce calcul est pondéré par l'état courant de l'aéronef 50, la phase de vol et les difficultés des tâches courantes de la mission

Selon un exemple, la valeur de comparaison *VC* est déterminée, lors de la quatrième étape 4, afin qu'elle soit égale au niveau fonctionnel courant du pilote *NFCP*.

Selon l'invention, au cours de la quatrième étape 4, l'ordinateur de bord 11 calcule une tendance *T* de l'évolution du niveau fonctionnel du pilote. Cette tendance *T* est représentée sur le graphe de la figure 2, par la droite 9, le temps t étant en abscisse et le niveau fonctionnel du pilote *NFP* en ordonnée. Cette droite 9 est définie afin d'être la plus proche des points $(NFP_i, t_i)$ et correspond à l'équation $T = a \times t + b$.

Cette droite 9 se caractérise par le fait qu'elle minimise la somme des distances au carré des points $(NFP_i, t_i)$ à cette droite 9. On peut en déduire, comme évoqué précédemment que

$$a = \frac{\sum_{i=1}^{n}\left[\left(t_i - t_{moyen}\right) \times \left(NFP_i - NFP_{moyen}\right)\right]}{\sum_{i=1}^{n}\left(t_i - t_{moyen}\right)^2} \text{ , sachant que } b = NFP_{moyen} - a \times t_{moyen}$$

Une fois la tendance *T* de l'évolution du niveau fonctionnel du pilote définie, on peut estimer un niveau fonctionnel prédictif du pilote *NFPP* selon la formule $NFPP = a \times (t_n + \Delta t) + b$, avec $\Delta t$ représentant la variation de temps à partir du temps $t_n$. La valeur de $\Delta t$ peut être de l'ordre d'une seconde à quelques secondes.

Ensuite, on détermine une valeur de comparaison VC afin qu'elle soit égale au niveau fonctionnel prédictif du pilote *NFPP.*

Lors de la cinquième étape 5, on compare cette valeur de comparaison *VC* avec les niveaux fonctionnels de référence *ValRef1*, *ValRef2* et *ValRef3*. Ces niveaux fonctionnels de référence *ValRef1*, *ValRef2* et *ValRef3* sont représentés sur le graphe de la figure 2 et correspondent à des niveaux fonctionnels limites du pilote à partir desquels il ne peut plus prendre pleinement conscience de la situation, ou bien prendre une décision adéquate ou bien réaliser efficacement la totalité des actions nécessaires.

Une aide doit alors lui être apportée afin de lui permettre d'assurer la continuité de la mission avec le maximum de sécurité. Cette aide lui est alors fournie par l'intermédiaire de l'IHM 20, au cours de la sixième étape 6, en fonction de cette valeur de comparaison *VC* et des trois niveaux fonctionnels de référence *ValRef1*, *ValRef2* et *ValRef3*, au minimum via des informations appropriées, voire par la réalisation, tout ou partie, de certaines tâches.

Tout d'abord, si la valeur de comparaison *VC* est inférieure aux niveaux fonctionnels de référence, aucune adaptation de l'IHM 20 n'est nécessaire. Dans ce cas, aucune modification de l'IHM 20 n'est effectuée, le pilote étant pleinement apte à réaliser les différentes tâches courantes de la phase de vol. Le procédé redémarre alors un cycle afin de déterminer une nouvelle valeur de comparaison *VC.*

Par contre, si la valeur de comparaison VC est égale ou supérieure à un premier niveau fonctionnel de référence *ValRef1* et inférieure à un second niveau fonctionnel de référence *ValRef2*, une adaptation de l'IHM 20 est nécessaire afin d'améliorer la prise de conscience de la situation par le pilote. Dans ce cas, au moins une modification de la présentation des informations fournies au pilote est effectuée. Le procédé redémarre ensuite un cycle afin de déterminer une nouvelle valeur de comparaison VC.

De plus, si la valeur de comparaison *VC* est égale ou supérieure à un second niveau fonctionnel de référence *ValRef2* et inférieure à un troisième niveau fonctionnel de référence *ValRef3*, une adaptation de l'IHM 20 est nécessaire afin d'assister le pilote dans sa prise de décision. Dans ce cas, une proposition de solution au problème courant à résoudre est indiquée au pilote. Le procédé redémarre ensuite un cycle afin de déterminer une nouvelle valeur de comparaison *VC.*

Enfin, si la valeur de comparaison *VC* est égale ou supérieure à un troisième niveau fonctionnel de référence *ValRef3*, une adaptation autoritaire de l'IHM 20 est indispensable pour agir à la place du pilote. Dans ce cas, une reprise automatique d'autorité par l'aéronef au détriment du pilote est engagée. Le procédé redémarre ensuite un cycle afin de déterminer une nouvelle valeur de comparaison *VC.*

On constate, dans l'exemple représenté sur le graphe de la figure 2 que le niveau fonctionnel du pilote *NFP* à l'instant $t_n$ est compris entre *ValRef1* et *ValRef2*, et que le niveau fonctionnel prédictif du pilote *NFPP* à l'instant $t_n + \Delta t$ est compris entre *ValRef2* et *ValRef3*. Ainsi lors de la sixième étape 6, l'IHM 20 proposera au pilote une solution au problème rencontré afin d'anticiper ce niveau fonctionnel prédictif *NFPP* et de l'aider à résoudre ce problème.

Naturellement, la présente invention est sujette à de nombreuses variations quant à sa mise en œuvre. Bien que plusieurs modes de réalisation aient été décrits, on comprend bien qu'il n'est pas concevable d'identifier de manière exhaustive tous les modes possibles. Il est bien sûr envisageable de remplacer un moyen décrit par un moyen équivalent sans sortir du cadre de la présente invention dont l'étendue de protection est définie par les revendications ci-jointes.

**Revendications**

1. Procédé d'adaptation d'une interface homme-machine (20) d'un aéronef (50),
   au cours duquel,

   - lors d'une première étape (1), on détermine, avant le démarrage d'une mission, des caractéristiques de ladite mission ainsi que des caractéristiques dudit pilote et son état physiologique initial,
   - lors d'une seconde étape (2), on détermine l'avancement de ladite mission et un état courant dudit aéronef (50) ainsi qu'un comportement courant dudit pilote au cours de ladite mission,
   - lors d'une troisième étape (3), on détermine un niveau fonctionnel courant dudit pilote *NFCP* en utilisant lesdites caractéristiques de ladite mission, lesdites caractéristiques et ledit état physiologique initial dudit pilote, ledit avancement de ladite mission, ledit état courant dudit aéronef (50) et ledit comportement courant dudit pilote,
- lors d'une quatrième étape (4), on détermine une valeur de comparaison $VC$ en fonction dudit niveau fonctionnel courant $NFCP,$

le procédé étant **caractérisé en ce que**:

- lors d'une cinquième étape (5), on compare ladite valeur de comparaison VC avec au moins un niveau fonctionnel de référence $ValRef,$ et
- lors d'une sixième étape (6), on agit sur ladite interface homme-machine (20) en fonction de ladite valeur de comparaison VC et de chaque niveau fonctionnel de référence $ValRef,$

et pour déterminer la valeur de comparaison VC, on enregistre ledit niveau fonctionnel courant $NFCP$ dans un historique desdits niveaux fonctionnels dudit pilote $NFP,$ puis on calcule une tendance $T$ d'une évolution dudit niveau fonctionnel $NFP$ à partir dudit historique, ensuite on détermine un niveau fonctionnel prédictif dudit pilote $NFPP,$ ladite valeur de comparaison $VC$ étant égale audit niveau fonctionnel prédictif $NFPP.$

2. Procédé selon la revendication 1,
**caractérisé en ce que** ledit procédé comporte trois niveaux fonctionnels de référence $ValRef1$, $ValRef2$ et $ValRef3$ afin que,

- si ladite valeur de comparaison VC est inférieure auxdits niveaux fonctionnels de référence $ValRef1$, $ValRef2$ et $ValRef3$, aucune modification de ladite interface homme-machine (20) n'est effectuée lors de ladite sixième étape (6),
- si ladite valeur de comparaison VC est égale ou supérieure au premier niveau fonctionnel de référence $ValRef1$ et inférieure au second niveau fonctionnel de référence $ValRef2$, au moins une modification de présentation des informations fournies audit pilote est effectuée lors de ladite sixième étape (6),
- si ladite valeur de comparaison VC est égale ou supérieure au second niveau fonctionnel de référence $ValRef2$ et inférieure au troisième niveau fonctionnel de référence $ValRef3$, une proposition de solution au problème courant à résoudre est indiquée audit pilote lors de ladite sixième étape (6),
- si ladite valeur de comparaison VC est égale ou supérieure au troisième niveau fonctionnel de référence $ValRef3$, une reprise automatique d'autorité par ledit aéronef au détriment dudit pilote est engagée lors de ladite sixième étape (6).

3. Procédé selon l'une quelconque des revendications 1 à 2, **caractérisé en ce que** lesdites caractéristiques de ladite mission comprennent un ensemble des différentes phases de vol de ladite mission et des tâches associées ainsi qu'un niveau de difficulté de chaque phase de vol et de chaque tâche.

4. Procédé selon l'une quelconque des revendications 1 à 3, **caractérisé en ce que** lesdites caractéristiques dudit pilote comprennent son niveau d'expérience et ses connaissances dans la réalisation de différentes tâches ainsi que son état psychologique.

5. Procédé selon l'une quelconque des revendications 1 à 4, **caractérisé en ce que** ledit comportement courant dudit pilote au cours de ladite mission comprend son état physiologique courant ainsi qu'une fréquence courante de ses erreurs.

6. Procédé selon l'une quelconque des revendications 1 à 5, **caractérisé en ce que** ladite interface homme-machine (20) utilise des systèmes neuronaux à apprentissage pour déterminer ledit niveau fonctionnel courant $NFCP$ lors de ladite troisième étape (3).

7. Procédé selon l'une quelconque des revendications 1 à 6, **caractérisé en ce que** ladite interface homme-machine (20) dudit aéronef (50) comporte au moins un système d'affichage d'informations (21), au moins un système d'information sonore (22), au moins un système d'information tactile (23) ou bien au moins un système de pilotage automatique (30) dudit aéronef (50).

8. Procédé selon l'une quelconque des revendications 1 à 7, **caractérisé en ce que** l'on détermine ledit niveau fonctionnel prédictif $NFPP$ selon la formule $NFPP = a \times (t_n + \Delta t) + b,$ avec $t_n$ représentant le temps du dernier niveau fonctionnel courant $NFCP_n$ déterminé et $\Delta t$ une variation de temps, les valeurs de $a$ et $b$ étant selon les formules suivantes,

$$a = \frac{\sum_{i=1}^{n}\left[\left(t_i - t_{moyen}\right) \times \left(NFP_i - NFP_{moyen}\right)\right]}{\sum_{i=1}^{n}\left(t_i - t_{moyen}\right)^2}, \quad b = NFP_{moyen} - a \times t_{moyen},$$

$NFP_i$ représentant ledit niveau fonctionnel dudit pilote correspondant au temps $t_i$, $NFP_{moyen}$ ledit niveau fonctionnel moyen dudit pilote sur l'intervalle de temps de $t_1$ à $t_n$ et $t_{moyen}$ le temps moyen sur ledit intervalle de temps tel que

$$NFP_{moyen} = \frac{\sum_{i=1}^{n} NFP_i}{n}$$

et

$$t_{moyen} = \frac{\sum_{i=1}^{n} t_i}{n}.$$

9. Dispositif d'adaptation (10) d'une interface homme-machine (20) d'un aéronef (50), ledit dispositif (10) comprenant

- un ensemble de capteurs physiologiques (12) mesurant un état physiologique dudit pilote,
- au moins un système scrutateur (13) contrôlant des actions dudit pilote afin de détecter d'éventuelles erreurs effectuées par ledit pilote déterminant ainsi une fréquence d'erreurs dudit pilote,
- au moins deux bases de données (14,15), une première base de données (14) contenant des caractéristiques de différentes missions réalisables et une seconde base de données (15) contenant des caractéristiques dudit pilote,
- au moins une unité de traitement (11), comprenant au moins une mémoire, au moins un processeur et apte à être relié à ladite interface homme-machine (20),

ladite unité de traitement (11) comportant un moyen de calcul et un moyen de stockage, ledit moyen de calcul exécutant des instructions stockées dans ledit moyen de stockage pour :

- déterminer, avant le démarrage d'une mission, des caractéristiques de ladite mission issues de ladite première base de données (14) ainsi que des caractéristiques dudit pilote issues de ladite seconde base de données (15) et un état physiologique initial dudit pilote,
- déterminer l'avancement de ladite mission et un état courant dudit aéronef (50) ainsi qu'un comportement courant dudit pilote au cours de ladite mission, ledit comportement courant dudit pilote comprenant son état physiologique courant et une fréquence courante de ses erreurs, ledit état courant dudit aéronef (50) étant obtenu par l'intermédiaire de différents capteurs présents dans ledit aéronef (50),
- déterminer un niveau fonctionnel courant dudit pilote *NFCP,* en utilisant lesdites caractéristiques de ladite mission, lesdites caractéristiques et ledit état physiologique initial dudit pilote, ledit avancement de ladite mission, ledit état courant dudit aéronef (50) et ledit comportement courant dudit pilote au cours de ladite mission,
- déterminer une valeur de comparaison VC en fonction dudit niveau fonctionnel courant NFCP,

le dispositif étant **caractérisé en ce que** l'unité de calcul (11) est apte à:

- comparer ladite valeur de comparaison VC avec au moins un niveau fonctionnel de référence *ValRef,* et
- agir sur ladite interface homme-machine (20) en fonction de ladite valeur de comparaison VC et de chaque niveau fonctionnel de référence *ValRef,*

le dispositif comprenant une troisième base de données (16) présente dans ledit aéronef (50) permettant de stocker lesdits niveaux fonctionnels dudit pilote *NFP,* constituant ainsi un historique desdits niveaux fonctionnels, ladite unité de traitement (11) calculant une tendance *T* d'une évolution dudit niveau fonctionnel *NFP* à partir dudit historique et déterminant un niveau fonctionnel prédictif *NFPP* basé sur ladite évolution *T,* ladite valeur de comparaison *VC* étant égale audit niveau fonctionnel prédictif NFPP.

**10.** Dispositif selon la revendication 9, ladite interface homme-machine (20) comportant au moins un système d'affichage d'informations (21), au moins un système d'information sonore (22), au moins un système d'information tactile (23) ou au moins un système de pilotage automatique (30) dudit aéronef (50), **caractérisé en ce que** ledit dispositif (10) comporte trois niveaux fonctionnels de référence *ValRef1*, *ValRef2* et *ValRef3* afin que,

- ladite unité de traitement (11) n'effectue aucune modification de ladite interface homme-machine (20) quand ladite valeur de comparaison VC est inférieure auxdits niveaux fonctionnels de référence *ValRef1*, *ValRef2* et *ValRef3,*
- ladite unité de traitement (11) effectue au moins une modification de présentation des informations fournies par ladite interface homme-machine (20) audit pilote quand ladite valeur de comparaison VC est égale ou supérieure premier niveau fonctionnel de référence *ValRef1* et inférieure au second niveau fonctionnel de référence *ValRef2,*
- ladite unité de traitement (11) indique audit pilote, par l'intermédiaire de ladite interface homme-machine (20), une proposition de solution au problème courant à résoudre quand ladite valeur de comparaison VC est égale ou supérieure au second niveau fonctionnel de référence *ValRef2* et inférieure au troisième niveau fonctionnel de référence *ValRef3,*
- ladite unité de traitement (11) effectue, par l'intermédiaire de ladite interface homme-machine (20), une reprise automatique d'autorité par ledit aéronef au détriment dudit pilote quand ladite valeur de comparaison VC est égale ou supérieure au troisième niveau fonctionnel de référence *ValRef3.*

**Patentansprüche**

**1.** Verfahren zur Anpassung einer Mensch-Maschine-Schnittstelle (20) eines Luftfahrzeugs (50), bei dem

- in einem ersten Schritt (1) vor dem Beginn einer Mission Eigenschaften der Mission sowie Eigenschaften des Piloten und sein physiologischer Ausgangszustand bestimmt werden,
- in einem zweiten Schritt (2) der Fortschritt der Mission und ein aktueller Status des Luftfahrzeugs (50) sowie ein aktuelles Verhalten des Piloten während der Mission bestimmt werden,
- in einem dritten Schritt (3) ein aktuelles Funktionsniveau NFCP des Piloten unter Verwendung der Eigenschaften der Mission, der Eigenschaften und des physiologischen Anfangszustands des Piloten, des Fortschritts der Mission, des aktuellen Zustands des Luftfahrzeugs (50) und des aktuellen Verhaltens des Piloten bestimmt wird,
- in einem vierten Schritt (4) in Abhängigkeit von dem genannten aktuellen Funktionsniveau NFCP ein Vergleichswert VC bestimmt wird,

wobei das Verfahren **dadurch gekennzeichnet ist, dass**:

- in einem fünften Schritt (5) der genannte Vergleichswert VC mit mindestens einem Referenz-Funktionsniveau ValRef verglichen wird, und
- in einem sechsten Schritt (6) in Abhängigkeit von dem Vergleichswert VC und jedem Referenz-Funktionsniveau ValRef auf die Mensch-Maschine-Schnittstelle (20) eingewirkt wird, und, um den Vergleichswert VC zu bestimmen, das aktuelle Funktionsniveau NFCP in einer Historie der Funktionsniveaus des Piloten NFP aufgezeichnet wird, dann ein Trend T einer Entwicklung des Funktionsniveaus NFP aus der Historie berechnet wird, und dann ein vorhergesagtes Funktionsniveau NFPP des Piloten bestimmt wird, wobei der Vergleichswert VC gleich dem vorhergesagten Funktionsniveau NFPP ist.

**2.** Verfahren nach Anspruch 1,
**dadurch gekennzeichnet, dass** das Verfahren drei ReferenzFunktionsniveaus ValRef1, ValRef2 und ValRef3 umfasst, so dass

- wenn der Vergleichswert VC niedriger ist als die ReferenzFunktionsniveaus ValRef1, ValRef2 und ValRef3, in dem sechsten Schritt (6) keine Änderung an der Mensch-Maschine-Schnittstelle (20) vorgenommen wird,

- wenn der Vergleichswert VC gleich oder höher als das erste Referenz-Funktionsniveau ValRef1 und niedriger als das zweite Referenz-Funktionsniveau ValRef2 ist, im sechsten Schritt (6) mindestens eine Änderung in der Darstellung der dem Piloten zur Verfügung gestellten Informationen vorgenommen wird,

- wenn der Vergleichswert VC gleich oder höher als das zweite Referenz-Funktionsniveau ValRef2 und niedriger als das dritte Referenz-Funktionsniveau ValRef3 ist, dem Piloten im sechsten Schritt (6) ein Lösungsvorschlag für das aktuelle zu lösende Problem angezeigt wird,

- wenn der Vergleichswert VC gleich oder höher als das dritte Referenz-Funktionsniveau ValRef3 ist, im sechsten Schritt (6) eine automatische Wiederaufnahme der Kontrolle durch das Luftfahrzeug zum Nachteil des Piloten eingeleitet wird.

3. Verfahren nach einem der Ansprüche 1 bis 2, **dadurch gekennzeichnet, dass** die Eigenschaften der Mission einen Satz der verschiedenen Flugphasen der Mission und der damit verbundenen Aufgaben sowie einen Schwierigkeitsgrad jeder Flugphase und jeder Aufgabe umfassen.

4. Verfahren nach einem der Ansprüche 1 bis 3, **dadurch gekennzeichnet, dass** zu den Eigenschaften des Piloten sein Erfahrungsund Wissensstand bei der Durchführung verschiedener Aufgaben sowie sein psychologischer Zustand gehören.

5. Verfahren nach einem der Ansprüche 1 bis 4,
**dadurch gekennzeichnet, dass** das aktuelle Verhalten des Piloten während der Mission seinen aktuellen physiologischen Zustand und eine aktuelle Häufigkeit seiner Fehler umfasst.

6. Verfahren nach einem der Ansprüche 1 bis 5,
**dadurch gekennzeichnet, dass** die Mensch-Maschine-Schnittstelle (20) lernende neuronale Systeme verwendet, um im dritten Schritt (3) das aktuelle Funktionsniveau NFCP zu bestimmen.

7. Verfahren nach einem der Ansprüche 1 bis 6,
**dadurch gekennzeichnet, dass** die Mensch-Maschine-Schnittstelle (20) des Luftfahrzeugs (50) mindestens ein Informationsanzeigesystem (21), mindestens ein Audioinformationssystem (22), mindestens ein taktiles Informationssystem (23) oder mindestens ein Autopilotsystem (30) des Luftfahrzeugs (50) umfasst.

8. Verfahren nach einem der Ansprüche 1 bis 7,
**dadurch gekennzeichnet, dass** das vorhergesagte Funktionsniveau NFPP gemäß der Formel

$$NFPP = a \times \left( t_n + \Delta t \right) + b$$

bestimmt wird, wobei $t_n$ die Zeit der letzten Bestimmung des aktuellen Funktionsniveaus $NFCP_n$ und $\Delta t$ eine Zeitveränderung darstellt, wobei die Werte von a und b folgenden Formeln genügen,

$$a = \frac{\sum_{i=1}^{n} \left[ \left( t_i - t_{moyen} \right) \times \left( NFP_i - NFP_{moyen} \right) \right]}{\sum_{i=1}^{n} \left( t_i - t_{moyen} \right)^2}, \quad b = NFP_{moyen} - a \times t_{moyen},$$

wobei $NFP_i$ das Funktionsniveau des Piloten zur Zeit $t_i$ darstellt, $NFP_{moyen}$ das durchschnittliche Funktionsniveau des Piloten über das Zeitintervall von $t_1$ bis $t_n$ und $t_{moyen}$ die durchschnittliche Zeit über das Zeitintervall angibt, so dass

$$NFP_{moyen} = \frac{\sum_{i=1}^{n} NFP_i}{n}$$

und

$$t_{moyen} = \frac{\sum_{i=1}^{n} t_i}{n},$$

9. Vorrichtung (10) zur Anpassung einer Mensch-Maschine-Schnittstelle (20) eines Luftfahrzeugs (50), wobei die Vorrichtung (10) umfasst:

- einen Satz physiologischer Sensoren (12), die einen physiologischen Zustand des Piloten messen,
- mindestens ein Erfassungssystem (13), das die Aktionen des Piloten überwacht, um mögliche Fehler des Piloten zu erkennen und so die Fehlerhäufigkeit des Piloten zu bestimmen,
- mindestens zwei Datenbanken (14, 15), wobei eine erste Datenbank (14) Eigenschaften verschiedener durchführbarer Missionen enthält und eine zweite Datenbank (15) Eigenschaften des genannten Piloten enthält,
- mindestens eine Verarbeitungseinheit (11), die mindestens einen Speicher und mindestens einen Prozessor umfasst und an die Mensch-Maschine-Schnittstelle (20) anschließbar ist,

wobei die Verarbeitungseinheit (11) eine Berechnungseinrichtung und eine Speichereinrichtung aufweist, wobei die Berechnungseinrichtung in der Speichereinrichtung gespeicherte Befehle ausführt, um:

- vor Beginn einer Mission Eigenschaften der Mission aus der ersten Datenbank (14) sowie Eigenschaften des Piloten aus der zweiten Datenbank (15) und einen physiologischen Anfangszustand des Piloten zu bestimmen,
- den Fortschritt der Mission und einen aktuellen Zustand des Luftfahrzeugs (50) sowie ein aktuelles Verhalten des Piloten während der Mission zu bestimmen, wobei das aktuelle Verhalten des Piloten seinen aktuellen physiologischen Zustand und eine aktuelle Häufigkeit seiner Fehler umfasst, wobei der aktuelle Zustand des Luftfahrzeugs (50) durch verschiedene in dem Luftfahrzeug (50) vorhandene Sensoren erhalten wird,
- ein aktuelles Funktionsniveau NFCP des Piloten zu bestimmen, wobei die Eigenschaften der Mission, die Eigenschaften und der physiologische Anfangszustand des Piloten, der Fortschritt der Mission, der aktuelle Zustand des Luftfahrzeugs (50) und das aktuelle Verhalten des Piloten während der Mission verwendet werden,
- in Abhängigkeit von dem genannten aktuellen Funktionsniveau NFCP einen Vergleichswert VC zu ermitteln,

wobei die Vorrichtung **dadurch gekennzeichnet ist, dass** die Recheneinheit (11) in der Lage ist:

- den Vergleichswert VC mit mindestens einem Referenz-Funktionsniveau ValRef zu vergleichen, und
- auf die Mensch-Maschine-Schnittstelle (20) in Abhängigkeit von dem genannten Vergleichswert VC und von jedem Referenz-Funktionsniveau ValRef einzuwirken,

wobei die Vorrichtung eine dritte Datenbank (16) umfasst, die in dem Luftfahrzeug (50) vorhanden ist, um die Funktionsniveaus NFP des Piloten zu speichern und somit eine Historie der Funktionsniveaus zu bilden, wobei die Verarbeitungseinheit (11) einen Trend T einer Entwicklung des Funktionsniveaus NFP ausgehend von der Historie berechnet und ein vorhergesagtes Funktionsniveau NFPP auf der Grundlage des Trends T bestimmt, wobei der Vergleichswert VC gleich dem vorhergesagten Funktionsniveau NFPP ist.

10. Vorrichtung nach Anspruch 9, bei der die Mensch-Maschine-Schnittstelle (20) mindestens ein Informationsanzeigesystem (21), mindestens ein Audioinformationssystem (22), mindestens ein taktiles Informationssystem (23) oder mindestens ein Autopilotsystem (30) des Luftfahrzeugs (50) umfasst,
**dadurch gekennzeichnet, dass** die Vorrichtung (10) drei Referenz-Funktionsniveaus ValRef1, ValRef2 und ValRef3 hat, so dass

- die Verarbeitungseinheit (11) keine Änderung an der Mensch-Maschine-Schnittstelle (20) vornimmt, wenn der Vergleichswert VC niedriger ist als die Referenzfunktionsniveaus ValRef1, ValRef2 und ValRef3,
- die Verarbeitungseinheit (11) mindestens eine Änderung in der Darstellung der von der Mensch-Maschine-Schnittstelle (20) an den Piloten gelieferten Informationen vornimmt, wenn der Vergleichswert VC gleich oder höher als das erste Referenzfunktionsniveau ValRef1 und niedriger als das zweite Referenzfunktionsniveau ValRef2 ist,
- die Verarbeitungseinheit (11) dem Piloten über die Mensch-Maschine-Schnittstelle (20) einen Lösungsvorschlag für das aktuelle zu lösende Problem anzeigt, wenn der Vergleichswert VC gleich oder höher als das zweite Referenz-Funktionsniveau ValRef2 und niedriger als das dritte Referenz-Funktionsniveau ValRef3 ist,
- die Verarbeitungseinheit (11) über die Mensch-Maschine-Schnittstelle (20) eine automatische Übernahme der Kontrolle über das Luftfahrzeug zum Nachteil des Piloten durchführt, wenn der Vergleichswert VC gleich oder höher als das dritte Referenz-Funktionsniveau ValRef3 ist.

**Claims**

1. Method for adapting a human-machine interface (20) of an aircraft (50), in which:

   - during a first step (1), before the start of a mission, characteristics of said mission and characteristics of a pilot and the pilot's initial physiological state are determined;
   - during a second step (2), the progress of said mission and a current state of said aircraft (50) and a current behaviour of said pilot during said mission are determined;
   - during a third step (3), a current functional level of said pilot *NFCP* is determined by using said characteristics of said mission, said characteristics and said initial physiological state of said pilot, said progress of said mission, said current state of said aircraft (50) and said current behaviour of said pilot;
   - during a fourth step (4), a comparison value VC is determined on the basis of said current functional level *NFCP;*

   the method being **characterised in that**:

   - during a fifth step (5), said comparison value VC is compared with at least one reference functional level *ValRef*; and
   - during a sixth step (6), said human-machine interface (20) is controlled on the basis of said comparison value VC and of each reference functional level *ValRef*;

   and, in order to determine the comparison value *VC,* said current functional level *NFCP* is recorded in a history of said functional levels of said pilot *NFP,* then a trend *T* of an evolution of said functional level *NFP* is calculated from said history, then a predictive functional level of said pilot *NFPP* is determined, said comparison value VC being equal to said predictive functional level *NFPP.*

2. Method according to claim 1, **characterised in that** said method comprises three reference functional levels *ValRef1, ValRef2* and *ValRef3* such that

   - if said comparison value VC is less than said reference functional levels *ValRef1, ValRef2* and *ValRef3*, a modification of said human-machine interface (20) is not carried out during said sixth step (6),
   - if said comparison value VC is greater than or equal to the first reference functional level *ValRef1* and less than the second reference functional level *ValRef2,* at least one modification of presentation of the information provided to said pilot is carried out during said sixth step (6),
   - if said comparison value VC is greater than or equal to the second reference functional level *ValRef2* and less than the third reference functional level *ValRef3,* a proposal for a solution to the current problem to be resolved is indicated to said pilot during said sixth step (6),
   - if said comparison value VC is greater than or equal to the third reference functional level *ValRef3,* an automatic takeover of authority by said aircraft to the detriment of said pilot is initiated during said sixth step (6).

3. Method according to either claim 1 or claim 2, **characterised in that** said characteristics of said mission include a set of different flight phases of said mission and associated tasks and a level of difficulty of each flight phase and each task.

4. Method according to any of claims 1 to 3, **characterised in that** said characteristics of said pilot include the pilot's level of experience and knowledge in performing various tasks and the pilot's psychological state.

5. Method according to any of claims 1 to 4, **characterised in that** said current behaviour of said pilot during said mission includes the pilot's current physiological state and a current frequency of the pilot's errors.

6. Method according to any of claims 1 to 5, **characterised in that** said human-machine interface (20) uses neural learning systems to determine said current functional level *NFCP* during said third step (3).

7. Method according to any of claims 1 to 6, **characterised in that** said human-machine interface (20) of said aircraft (50) comprises at least one information display system (21), at least one sound information system (22), at least one tactile information system (23) or at least one autopilot system (30) of said aircraft (50).

8. Method according to any of claims 1 to 7, **characterised in that** said predictive functional level *NFPP* is determined according to the formula $NFPP = a \times (t_n + \Delta t) + b$, where $t_n$ represents the determined time of the last current functional level $NFCP_n$ and $\Delta t$ represents a time variation, the values of a and b being according to the following formulas,

$$a = \frac{\sum_{i=1}^{n}[(t_i - t_{moyen}) \times (NFP_i - NFP_{moyen})]}{\sum_{i=1}^{n}(t_i - t_{moyen})^2}, b = NFP_{moyen} - a \times t_{moyen},$$

$NFP_i$ representing said functional level of said pilot corresponding to the time $t_i$, $NFP_{moyen}$ representing said mean functional level of said pilot over the time interval from $t_1$ to $t_n$, and $t_{moyen}$ representing the mean time over said time interval such as $NFP_{moyen} = \frac{\sum_{i=1}^{n} NFP_i}{n}$ and $t_{moyen} = \frac{\sum_{i=1}^{n} t_i}{n}$.

9. Device (10) for adapting a human-machine interface (20) of an aircraft (50), said device (10) comprising

   - a set of physiological sensors (12) measuring a physiological state of said pilot,
   - at least one scanning system (13) which monitors actions of said pilot in order to detect possible errors made by said pilot, thus determining an error frequency of said pilot,
   - at least two databases (14, 15), a first database (14) containing characteristics of different feasible missions and a second database (15) containing characteristics of said pilot,
   - at least one processing unit (11), comprising at least one memory, at least one processor and being capable of being connected to said human-machine interface (20),

   said processing unit (11) comprising a calculation means and a storage means, said calculation means executing instructions stored in said storage means for:

   - determining, before the start of a mission, characteristics of said mission from said first database (14) and characteristics of said pilot from said second database (15) and an initial physiological state of said pilot;
   - determining the progress of said mission and a current state of said aircraft (50) and a current behaviour of said pilot during said mission, said current behaviour of said pilot including the pilot's current physiological state and a current frequency of the pilot's errors, said current state of said aircraft (50) being obtained by means of various sensors present in said aircraft (50);
   - determining a current functional level of said pilot *NFCP* by using said characteristics of said mission, said characteristics and said initial physiological state of said pilot, said progress of said mission, said current state of said aircraft (50) and said current behaviour of said pilot during said mission;
   - determining a comparison value VC on the basis of said current functional level *NFCP*;

   the device being **characterised in that** the calculation unit (11) is capable of:

   - comparing said comparison value VC with at least one reference functional level *ValRef*; and
   - controlling said human-machine interface (20) on the basis of said comparison value VC and of each reference functional level *ValRef,*

   the device comprising a third database (16) in said aircraft (50) for storing said functional levels of said pilot *NFP,* thus constituting a history of said functional levels, said processing unit (11) calculating a trend *T* of an evolution of said functional level *NFP* from said history and determining a predictive functional level *NFPP* based on said evolution *T,* said comparison value VC being equal to said predictive functional level *NFPP.*

10. Device according to claim 9, said human-machine interface (20) comprising at least one information display system (21), at least one sound information system (22), at least one tactile information system (23) or at least one autopilot system (30) of said aircraft (50), **characterised in that** said device (10) comprises three reference functional levels *ValRef1, ValRef2* and *ValRef3* such that

- said processing unit (11) does not carry out a modification of said human-machine interface (20) when said comparison value VC is less than said reference functional levels *ValRef1*, *ValRef2* and *ValRef3*,
- said processing unit (11) carries out at least one modification of presentation of the information provided by said human-machine interface (20) to said pilot when said comparison value VC is greater than or equal to the first reference functional level *ValRef1* and less than the second reference functional level *ValRef2*,
- said processing unit (11) indicates to said pilot, by means of said human-machine interface (20), a proposal for a solution to the current problem to be resolved when said comparison value VC is greater than or equal to the second reference functional level *ValRef2* and less than the third reference functional level *ValRef3*,
- said processing unit (11) carries out, by means of said human-machine interface (20), an automatic takeover of authority by said aircraft to the detriment of said pilot when said comparison value VC is greater than or equal to the third reference functional level *ValRef3*.

Fig.2

Fig.1

Fig.3

Fig.4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2888967
- EP 0292381 A
- US 20070063854 A
- EP 1975783 A

**Littérature non-brevet citée dans la description**

- Situation Awareness Modeling and Pilot State Estimation for tactical Cockpit Interfaces. *HIC International Conférence,* Août 1997
- An Approch to developing adaptative interface technology for advanced airborne crew stations. *Daytona Section Symposium,* 09 Avril 1997
- **DURIC Z et al.** Integrating perceptual and cognitive modeling for adapatative and intelligent human-computer interaction. *PROCEEDINGS OF THE IEEE,* 01 Juillet 2002, vol. 90